# EUROPÄISCHE PATENTSCHRIFT

(11) **EP 0 973 695 B1**
(45) Veröffentlichungstag und Bekanntmachung des Hinweises auf die Patenterteilung: **28.06.2006**
(21) Anmeldenummer: 98952733.8
(22) Anmeldetag: 22.10.1998
(51) Int. Cl.: C02F 11/04, C12M 1/107

(54) **BIOGASANLAGE**
BIOGAS FACILITY
INSTALLATION A BIOGAZ

(30) Priorität: 22.10.1997 DE 19746636
(43) Veröffentlichungstag der Anmeldung: 26.01.2000
(73) Patentinhaber: Von Nordenskjöld, Reinhart, Dr.-Ing., D-85658 Egmating-Münster (DE)
(72) Erfinder: Von Nordenskjöld, Reinhart, Dr.-Ing., D-85658 Egmating-Münster (DE)
(74) Vertreter: von Füner, Nicolai
(86) Internationale Anmeldenummer: PCT/EP1998/006702
(87) Internationale Veröffentlichungsnummer: WO 1999/020573

(56) Entgegenhaltungen:
- EP-A- 0 158 213
- WO-A-84/00038
- DE-A- 3 232 530
- DE-C- 19 538 579
- US-A- 4 594 078

## Beschreibung

Die Erfindung betrifft eine Anlage zur Erzeugung von Biogas durch anaeroben Abbau von organischem Material.

Biogas ist die Bezeichnung für ein mit Wasserdampf gesättigtes Gasgemisch, das bei der anaeroben Fermentation organischer Stoffe auf natürlichem Wege, z.B. in Seen oder Sümpfen, und auf verfahrenstechnischem Wege in einer Biogasanlage entsteht. Es besteht zu 55-75% aus Methan, zu 24-44% aus Kohlendioxid und in Spuren aus anderen Beimengungen. Biogas kann durch eine Verbrennung energetisch genutzt werden, wobei der Heizwert im Durchschnitt 21,5 x 10⁶ J/m3 (Streubreite: 14,0 bis 29,9 x 10⁶ J/m³) beträgt. Als Ausgangsstoffe für die Biogasgewinnung können grundsätzlich alle anaerob abbaubaren organischen Stoffe dienen. Bevorzugt sind jedoch Reststoffe, z.B. Flüssig-und Festmist, Klärschlämme und organisch hochbelastete Abwässer, organische Anteile des Hausmülls und Rohstoffe der Landwirtschaft und Landschaftspflege (u.a. Grüngut). Beim anaeroben Abbau der Ausgangsstoffe werden zunächst u.a. hochmolekulare Verbindungen in niedermolekulare Verbindungen zerlegt, sowie Fette zu Fettsäuren und Glycerin hydrolysiert. In der methanogenen Phase erfolgt dann die Umwandlung der entstandenen Verbindungen zu Kohlendioxid und Methan. Zusätzlich bilden sich aus dem entstandenen Kohlendioxid und dem freiwerdenden Wasserstoff neben Wasser weitere Mengen an Methan.

Biogasanlage ist die Bezeichnung für eine Anlage zur verfahrenstechnischen Umsetzung der anaeroben Fermentation zur Erzeugung von Biogas oder zur Reinigung von hochbelasteten Abwässern.

Aus gwf-Gas/Erdgas 124, Nr. 8, 389-394 (1983) ist eine Biogasanlage bekannt, die einen Vorbehälter für organische Rückstände, eine Beschickungseinrichtung, die mit einer Pumpe (eventuell mit Schneidewerk) und einer Heizung ausgestattet ist, einen Faulbehälter, der mit einem Rührwerk und einer Bodenheizung ausgestattet ist, eine Einrichtung zur Entnahme von ausgefaultem Biodung aus dem Faulbehälter in einen weiteren Behälter, sowie eine Einrichtung zur Entnahme des entstandenen Biogases aus dem Faulbehälter in einen Gasspeicher umfaßt. Bei dieser Biogasanlage sind die einzelnen Baugruppen, insbesondere Vorbehälter und Faulbehälter, separat im Freien angeordnet. Diese Biogasanlage kann bei Gärtemperaturen von 35 oder 55° C (im Faulbehälter) betrieben werden.

Bekannt ist ferner eine Biogasanlage der Firma Novatech Gesellschaft für umweltschonende Technologie mbH, Vellberg, die aus folgenden Baugruppen besteht: Aufbereitung, bestehend aus Vorgrube mit Homogenisier- und Pumpeinrichtung, sowie Feststoff-Dosierstation und Jauchesammelschacht; Gärbehälter mit Heizung, Wärmedämmung, Rührwerk und Gasdom; Austrag mit Separierung; Gasgruppe, bestehend aus Gasleitung, Entschwefelungseinrichtung, Gasspeicher und Armaturen; und Gasverwertung, bestehend aus Blockheizkraftwerk, Wärmespeicher und Verrohrung mit Armaturen. Auch bei dieser Biogasanlage sind die einzelnen Baugruppen jeweils separat im Freien angeordnet.

Bekannt ist ferner die Speicherbiogasanlage der Biogasgruppe im Bundschuh, die aus einer Vorgrube, einem Güllezulauf, einem Faulbehälter, dessen Decke mit einer Isolierschicht und einer Folienabdeckung ausgestattet ist, Einrichtungen zur Entnahme von Gas und Gülle aus dem Faulbehälter, sowie einem Kraft-Wärme-Aggregat besteht. Die Außenwände des Faulbehälters sind darüber hinaus mit einer Isolierschicht ausgestattet.

DE-19538579 beschreibt eine Anlage zur Herstellung von Biogas aus organischen Stoffen mit einem gasdichten Reaktor mit Gülleeinlauf, Gülleauslauf und Hiogasaustritt, einem asynchron und reversierend antreibbaren Rührwerk im Reaktor zum Durchmischen der Gülle und zugesetzter organischer Reststoffe bzw. reinen Reststoffmedien sowie einem Wärmetauscher im Reaktor zum Vorwärmen der eingegebenen Medien, wobei der Reaktor aus einem mit einem gemeinsamen Deckel verschlossenen äußeren zylindrischen Behälter und konzentrisch dazu angeordneten, im Durchmesser kleineren inneren zylindischen Behälter besteht, wobei beide Behälter durch eine Trennwand einseitig von.der Mitte aus gesehen getrennt sind und an der Vorderseite der Trennwand der Wärmetauscher mit Gülleeinlauf und Überlauf angeordnet sind und annähernd vor dem Wärmetauscher am Reaktorboden der Gülleauslauf mit Auslauftrichter vorhanden sind und an der Rückseite der Trennwand am Reaktorboden die Umlaufleitung mit aufgesetztem Umlauftrichter und darüber am oberen Rand des inneren Behälters ein Ausschnitt vorhanden sind und das Rührwerk durch einen im Deckel konzentrisch drehbar gelagerten angetriebenen Rührarm mit einem an seinem Ende angebrachten offenen Doppelknierohr gebildet ist, das sich durch das Medium bewegt, wobei sich im unteren Knie ein Flügelrührer mit darüberliegendem Schneidwerk und Gegenschneiden vorgesehen sind und der Flügelrührer und das Schneidwerk durch einen auf dem unteren Knie in der Gülle sitzenden Motor angetrieben werden und Rohrleitungen zum Einbringen von Zusatzstoffen oder Luft in den Reaktor und eine Absaugrohrleitung in den Reaktor münden.

Die US-PS 4,594,078 betrifft eine Anlage zur Produktion von Biomethan aus organischem Material, wobei der obere Teil des Gärbehälters sowie seine zentrale Tragesäule, an der dieser mechanisch befestigt ist, um die vertikale Mittelachse des Bottichs rotieren kann, der untere Teil des Gärbehälters mittels Rollen an der Oberseite der Seitenwände und der zentralen Säule gestützt wird, wobei diese zentrale Säule auf dem Bottichboden mittels eines Fußlagers gestützt wird, der mittlere Teil des Bottichs durch eine als Primärkammer bezeichnete zylindrische Kammer eingenommen (gebildet) wird, deren Achse identisch ist mit der Achse des Bottichs des Gärbehälters, eine unbewegliche Trennwand durch den Bottich führt und längs des Bottichradius angeordnet ist, eine starr mit dem oberen Teil des Gärbehälters und der-zentralen Säule verbundene bewegliche Trennwand kreisförmige Hin- und Herbewegungen ausführt, um dadurch den gesamten Bottichinhalt außerhalb der Primärkammer wegzukehren, und eine Umwälzpumpe und eine Förderpumpe am oberen Teil der beweglichen Trennwand angebracht sind. Während des Verschiebens der beweglichen Trennwand fördert die Umwälzpumpe - beiderseits der Trennwand - den gesamten während des Verschiebevorgangs weggekehrten Inhalt, und die Förderpumpe führt gleichzeitig die vom Boden der Primärkammer kommenden Materialien dem Strom der unter der Wirkung der Umwälzpumpe verschobenen Materialien zu. Das Biogas wird durch einen am beweglichen oberen Teil des Gärbehälters angebrachten flexiblen Tank gesammelt.

Im Hinblick auf den Stand der Technik lag der Erfindung die Aufgabe zugrunde, eine Biogasanlage bereitzustellen, die eine verbesserte Energiebilanz, eine wirtschaftliche Kompaktbauweise und die Erzeugung von Biogas mit reduziertem Anteil an Kohlendioxid und anderen Beimengungen gewährleisten.

Diese Aufgabe wird durch eine Biogasanlage mit den Merkmalen des Patentanspruchs 1 gelöst.

Die Unteransprüche betreffen bevorzugte Ausführungsformen der Biogasanlage von Patentanspruch 1.

Ein Vorteil der Erfindung besteht darin, daß in äußerst umweltfreundlicher Weise die Verbesserung der Energiebilanz über eine Verbesserung der Isolierung des Reaktionsbehälters durch geschickte Anordnung der einzelnen Baugruppen einer Biogasanlage zueinander bei gleichzeitiger Einsparung von reinem Isolieraufwand bzw. durch eine geschickte Produktführung herbeigeführt wird.

Die chemische Zusammensetzung von Biogas ist temperaturabhängig, wobei bei geringeren Temperaturen der Anteil an insbesondere CO₂ und H₂O im Biogas abnimmt. Die Erfindung nutzt in vorteilhafter Weise diesen Umstand durch entsprechende Wahl der Betriebstemperatur und durch Anordnung eines Bereiches zur Nachgasung des organischen Materials im geschlossenen Reaktorgehäuse aus.

Im Sinne der Erfindung bezeichnet "organisches Material" organisches Material, das im wesentlichen aus festen Bestandteilen besteht, z.B. Festmist und Grüngut. "Frisches organisches Material" bezeichnet organisches Material, bei dem im wesentlichen noch kein anaerober Abbau eingetreten ist. "Vorgesäuertes organisches Material" bezeichnet organisches Material in einem Stadium, in dem im wesentlichen noch kein Methan gebildet wird. "Gasendes organisches Material" bezeichnet organisches Material, das zumindest teilweise noch Methangas bildet. "Ausgefaultes organisches Material" bezeichnet organisches Material, das anaerob weitestgehend abgebaut ist.

Ein Ausführungsbeispiel der Erfindung wird nachstehend anhand von Zeichnungen näher erläutert. Es zeigt:
Fig. 1 eine schematische Draufsicht auf eine Biogasanlage; und
Fig. 2 eine Schnittdarstellung der Linie I-I der Fig. 1.

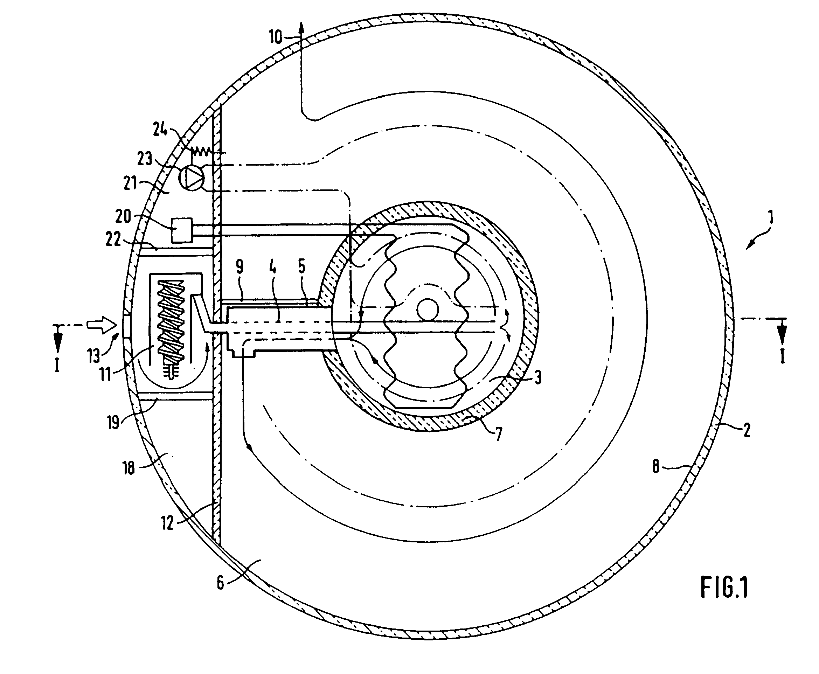

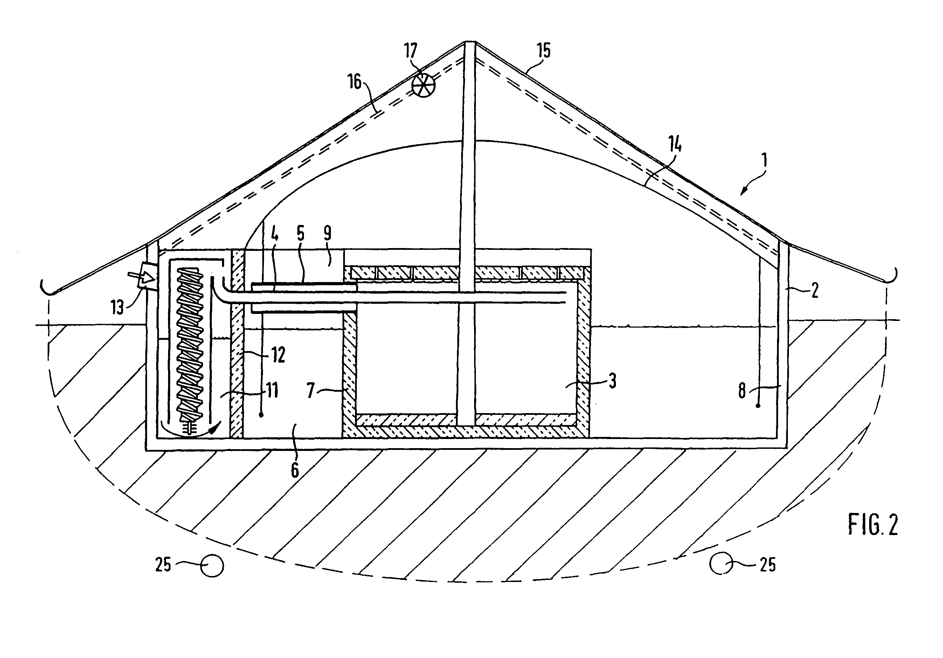

Die in den Figuren 1 und 2 dargestellte Biogasanlage 1 ist mit ihrem Vielfach-Isolierschutz insbesondere zum Betrieb in kälteren Klimazonen (bei Außentemperaturen von - 25° (Winter) bis + 25° C (Sommer)) geeignet.

Diese Biogasanlage weist ein im wesentlichen zylindrisches, geschlossenes Reaktorgehäuse 2 auf. In dem Reaktorgehäuse 2 ist ein an die Innenwandfläche 8 angrenzender Misch-, Fermentations- und Vorversorgungsbehälter 11 (Innentemperatur ca. 5 bis 10° C) vorgesehen, der durch eine Zwischenwand 12 von dem Zwischenbereich 6 abgetrennt ist und mit einer Beschickungseinrichtung 13 für frisches organisches Material und der Zufuhreinrichtung 4 in Verbindung steht.

Zum Betrieb der Biogasanlage 1 wird zunächst der Misch-, Fermentations- und Vorversorgungsbehälter 11 über die Beschickungseinrichtung 13 mit frischem organischem Material beschickt. Im Misch-, Fermentations- und Vorversorgungsbehälter 11 wird das frische organische Material einer im wesentlichen anaeroben Fermentation unterworfen und das erhaltene vorgesäuerte, organische Material mit Hilfe einer Kreislaufschnecke (mit integriertem Schneidwerk) zerkleinert und bis zu einer Stelle des Misch-, Fermentations- und Vorversorgungsbehälters 11 gefördert, die mit der Zufuhreinrichtung 4 in Verbindung steht und ferner so ausgewählt ist, daß die weitere Produktführung in der Biogasanlage 1 weitestgehend im freien Gefälle erfolgen kann. Der Misch-, Fermentations- und Vorversorgungsbehälter 11 kann alternativ auch mit einer geeigneten Pumpe und gegebenenfalls, sofern dies im Hinblick auf das verwendete frische organische Material notwendig ist, mit einem geeigneten Schneidewerk ausgestattet werden. Der Misch-, Fermentations- und Vorversorgungsbehälter 11 kann ferner mit einem Rührwerk ausgestattet sein.

Der Misch-, Fermentations- und Vorversorgungsbehälter 11 steht darüber hinaus mit Vorrichtungen zur pH-Kontrolle und Belüftung des vorgesäuerten, organischen Materials in Verbindung. Diese Vorrichtungen (nicht gezeigt) sind in einem Reserveraum 18 angeordnet, der in dem Reaktorgehäuse 2 an die Innenwandfläche 8 angrenzt und durch die Zwischenwand 12 von dem Zwischenbereich 6 und durch eine Wand 19 von dem Misch-, Fermentations- und Vorversorgungsbehälter 11 abgetrennt ist. Die Belüftung erlaubt im übrigen eine Erwärmung des vorgesäuerten, organischen Materials (thermophiler Bereich), wodurch die Heizungskosten für den Reaktionsbehälter 3 weiter reduziert werden können.

Das vorgesäuerte, organische Material gelangt nun über die Zufuhreinrichtung 4 in den im wesentlichen zylindrischen Reaktionsbehälter 3, der im thermischen Zentrum des geschlossenen Reaktorgehäuses 2 angeordnet ist. Im Sinne der Erfindung wird unter einer Anordnung im "thermischen Zentrum" eine zentrale Anordnung verstanden, die sich aber nicht rein geometrisch ergibt, sondern daraus, daß das Isolierpotential zur kalten Außentemperatur überall gleich ist und somit die Summe der Wärmeverluste minimiert wird.

Am Boden des Reaktionsbehälters 3 ist eine Heizeinrichtung, z.B. Heizspiralen, vorgesehen, die im wesentlichen zum Anfahren der Biogasanlage, d.h. zum Aufheizen des organischen Materials im Reaktionsbehälter 3 auf Gärtemperaturen von 25 bis 40° C oder (bei entsprechendem Rohmaterial) von 50 bis 60° C dient. Bevorzugt wird die Biogasanlage bei einer Gärtemperatur von 25 bis 35°C betrieben, da bei dieser Temperatur der Anteil an Kohlendioxid und anderen Beimengungen im Biogas gering ist, ohne daß die absolute Methanmenge wesentlich niedriger liegt als bei höheren Temperaturen. Die am Boden des Reaktionsbehälters 3 vorgesehene Heizeinrichtung ist an ein Blockheizkraftwerk 20 angeschlossen, das in dem Heizungsraum 21 angeordnet ist. Der Heizungsraum 21 grenzt im Reaktorgehäuse 2 an die Innenwandfläche 8 an und ist durch die Zwischenwand 12 von dem Zwischenbereich 6 und durch eine Wand 22 von dem Misch-, Fermentations- und Vorversorgungsbehälter 11 abgetrennt. Anstelle oder zusätzlich zu dieser Heizeinrichtung kann im Boden des Reaktionsbehälters 3 ein Heizungs- und Umwälzgassystem vorgesehen sein.

Am Boden (unterhalb der Heizeinrichtung) und in der Seitenwand des Reaktionsbehälters 3 ist eine Isolierschicht vorgesehen. Ferner ist im Reaktionsbehälter 3 oberhalb des organischen Materials eine Abdeckisolierung vorgesehen. Die Abdeckisolierung, die auf dem organischen Material schwimmend oder auf einem aufgespannten Netz angeordnet sein kann, trägt zu einer Reduzierung des H₂O-Anteils im Biogas bei.

An den oberen Bereich der Seitenwand des Reaktionsbehälters 3 ist eine Abfuhreinrichtung 5 für eine Abfuhr von nachgasendem, organischem Material aus dem Reaktionsbehälter 3 angeschlossen, wobei die Zufuhreinrichtung 4 zumindest teilweise als inneres Rohr eines Doppelrohrs durch die Abfuhreinrichtung 5 des Reaktionsbehälters 3 hindurchgeht. Ferner liegen der Anschluß der Abfuhreinrichtung 5 an den Reaktionsbehälter 3 und die Mündung der Zufuhreinrichtung 4 in den Reaktionsbehälter 3 im wesentlichen diametral bezüglich der Mittelachse des Reaktionsbehälters 3 gegenüber, um eine ausreichende mittlere Verweilzeit des organischen Materials im Reaktionsbehälter zu gewährleisten.

Die Abfuhreinrichtung 5 besitzt eine bodenwärts gekrümmte Öffnung, die so angeordnet ist, daß das nachgasende, organische Material aus dem Reaktionsbehälter 3 auf der der Auslaßeinrichtung 10 abgewandten Seite der Trennwand 9, die sich im wesentlichen parallel zu der Zufuhreinrichtung 4 und der Abfuhreinrichtung 5 zwischen der Außenwandfläche 7 des Reaktionsbehälters 3 und der Zwischenwand 12 erstreckt, in den im wesentlichen kreisförmigen Zwischenbereich 6 gelangt.

Der sich zwischen der Außenwandfläche 7 des Reaktionsbehälters 3 und der Innenwandfläche 8 des Reaktorgehäuses 2 bzw. der Zwischenwand 12 erstreckende Zwischenbereich 6 dient im wesentlichen der Nachgasung und Lagerung des aus dem Reaktionsbehälter 3 geführten organischen Materials. Das nachgasende organische Material wird über die Zeit nach Eintritt in den Zwischenbereich 6 fast vollständig um den Reaktionsbehälter 3 herum zur Auslaßeinrichtung 10 geführt. Die Temperatur des nachgasenden organischen Materials beträgt, sofern die Biogasanlage bei einer Gärtemperatur von ca. 30° C betrieben wird, unmittelbar nach Eintritt in den Zwischenbereich 6 ca. 20° C und sinkt, nachdem ungefähr die Hälfte des Zwischenbereichs 6 durchlaufen ist, auf Werte von ca. 10 bis 15° C. Ungefähr 20% des insgesamt in der Biogasanlage gewonnenen Methans geht auf die besonders methanreiche Nachgasung im Zwischenbereich 6 zurück. Das hinter der Auslaßeinrichtung 10 entnommene, ausgefaulte organische Material kann als Dünger verwendet werden.

Am Boden des Zwischenbereichs 6 ist alternativ ein Heizungs-und Umwälzsystem vorgesehen, das aus im Boden verlegten Ausblasrohren für mehr oder weniger erwärmtes Gas besteht, die an einen Umwälzventilator 23 und einen Gaserwärmer 24 angeschlossen sind, so daß in besonders vorteilhafter Weise "von innen heraus" beheizt wird. Der Umwälzventilator 23 und der Gaserwärmer 24 sind im Heizungsraum 21 angeordnet. Aufgrund der Anordnung der Aufheizeinrichtung im geschlossenen Reaktorgehäuse wird die Minimierung der Wärmeverluste weiter vorangetrieben. In diesem Zusammenhang wird darauf hingewiesen, daß ein derartiges Heizungs- und Umwälzgassystem alternativ oder kumulativ zu der oben beschriebenen Heizeinrichtung zum Aufheizen des Reaktionsbehälters 3 verwendet werden kann.

In dem geschlossenen Reaktorgehäuse 2 ist eine sich über den Reaktionsbehälter 3 und den Zwischenbereich 6 erstreckende Folie 14 angeordnet, um diese Teile des Reaktorgehäuses 2 nach oben gasdicht abzuschließen. Die Anordnung des durch die Folie 14 ausgebildeten Gasspeichers oberhalb des Reaktionsbehälters 3 und des Zwischenbereichs 6 trägt weiterhin aufgrund der Isolierwirkung des darin enthaltenen Gases zur Reduzierung der Heizungskosten für den Reaktionsbehälter 3 bei, wobei die Gastemperatur im Gasspeicher bei einer Gärtemperatur von ca. 30° C ca. 20° C beträgt. Im übrigen kann die Bewegung der Folie 14, d.h. ihr Weg, den sie aufgrund verschiedenem Füllungsgrad durchläuft, zur Gasmengenanzeige herangezogen werden.

Das geschlossene Reaktorgehäuse 2 ist nach oben durch ein Dach 15 abgeschlossen, wobei unterhalb des Dachs 15 eine Isolierzwischendecke 16 (mit einem Ventilator 17) im Abstand zum Dach 15 angeordnet ist. Bei einer derartigen Ausgestaltung des Dachbereichs des geschlossenen Reaktorgehäuses 2 wird über die Luftzone oberhalb der Folie 14 und die Luftzone zwischen der Isolierzwischendecke 16 und dem Dach 15 Sonnenenergie zur Verbesserung der Energiebilanz herangezogen, da selbst bei Minustemperaturen Einstrahlungen höhere Temperaturen unter dem Dach 15 erzeugen. Insgesamt stellt die Anordnung für die Gesamtanlage nach oben hin einen weiteren, zweischichtigen, wirkungsvollen Isolierbereich dar.

Das geschlossene Reaktorgehäuse 2 ist zumeist mehr oder weniger in das Erdreich eingelassen. Das geschlossene Reaktorgehäuse 2 kann jedoch auch durch einen Erdwall umhüllt werden. Im Erdreich unterhalb des geschlossenen Reaktorgehäuses 2 sind ferner Drainagen 25 angeordnet. Das Dach 15 des geschlossenen Reaktorgehäuses 2 erstreckt sich fast bis zur Oberfläche des das geschlossene Reaktorgehäuse 2 umgebenden Erdreichs, d.h. es ragt ringförmig deutlich über die Seitenwände des Reaktorgehäuses 2 hinaus, und wird durch eine Regenrinne abgeschlossen. Durch diese Maßnahmen wird sichergestellt, daß das umgebende Erdreich trocken ist und somit als gutes Isoliermedium dienen kann.

Die vorangehende Beschreibung zeigt, daß die Erfindung eine Biogasanlage betrifft, in der in völlig neuer und (bezüglich Investition und Betrieb) sehr wirtschaftlicher Weise alle Komponenten vorteilhaft und kompakt integriert sind, und die insbesondere für kühlere Klimazonen geeignet ist.

## Patentansprüche

1. Biogasanlage mit
- einem geschlossenen Reaktorgehäuse (2),
- einem in dem Reaktorgehäuse (2) an die Innenwandfläche (8) angrenzenden Misch-, Fermentations- und Vorversorgungsbehälter (11), der durch eine Zwischenwand (12) von dem Zwischenbereich (6) abgetrennt ist und mit einer Beschickungseinrichtung (13) für frisches organisches Material und einer Zufuhreinrichtung (4) in Verbindung steht,
- einem in dem Reaktorgehäuse (2) im Abstand zu dessen Innenwandfläche (8) angeordneten Reaktionsbehälter (3), an den eine Zufuhreinrichtung (4) für vorgesäuertes, organisches Material mündet und an den eine Abfuhreinrichtung (5) für eine Abfuhr von gasendem, organischem Material aus dem Reaktionsbehälter (3) angeschlossen ist,
- einer sich zwischen der Außenwandfläche (7) des Reaktionsbehälters (3) und der Innenwandfläche (8) des Reaktorgehäuses (2) erstreckenden Trennwand (9), und
- einer im Reaktionsbehälter (3) vorgesehenen Heizeinrichtung, wobei
- die Abfuhreinrichtung (5) so ausgebildet ist, dass sie das gasende, organische Material aus dem Reaktionsbehälter (3) auf einer Seite der Trennwand (9) in den Zwischenbereich (6) zwischen der Außenwandfläche (7) des Reaktionsbehälters (3) und der Innenwandfläche (8) des Reaktorgehäuses (2) einbringt,
- die Zufuhreinrichtung (4) und die Abfuhreinrichtung (5) des Reaktionsbehälters (3) rohrförmig ausgebildet sind, wobei die Zufuhreinrichtung (4) mit radialem Abstand durch die Abfuhreinrichtung (5) hindurchgeht, und
- im Reaktorgehäuse (2) in dem Bereich, der an die andere Seite der Trennwand (9) angrenzt, eine Auslasseinrichtung (10) für ausgefaultes, organisches Material vorgesehen ist.

2. Biogasanlage nach Anspruch 1, **dadurch gekennzeichnet, dass** der Misch-, Fermentations- und Vorversorgungsbehälter (11) mit einer Kreislaufschnecke ausgestattet ist.

3. Biogasanlage nach Anspruch 1 oder 2, **dadurch gekennzeichnet, dass** der Anschluss der Abfuhreinrichtung (5) an den Reaktionsbehälter (3) und die Mündung der Zufuhreinrichtung (4) in den Reaktionsbehälter (3) im wesentlichen diametral bezüglich der Mittelachse des Reaktionsbehälters (3) gegenüber liegen;

4. Biogasanlage nach einem der Ansprüche 1 bis 3, **dadurch gekennzeichnet, dass** in dem geschlossenen Reaktorgehäuse (2) eine gasdichte, sich über den Reaktionsbehälter (3) und den Zwischenbereich (6) erstreckende Folie (14) angeordnet ist.

5. Biogasanlage nach einem der Ansprüche 1 bis 4, **dadurch gekennzeichnet, dass** das geschlossene Reaktorgehäuse (2) nach oben durch ein Dach (15) abgeschlossen ist und unterhalb des Dachs (15) eine Isolierzwischendecke (16) im Abstand zum Dach (15) angeordnet ist.

6. Biogasanlage nach Anspruch 5, **dadurch gekennzeichnet, dass** in der Isolierzwischendecke (16) ein Ventilator (17) angeordnet ist.

7. Biogasanlage nach Anspruch 5 oder 6, **dadurch gekennzeichnet, dass** sich das Dach (15) des geschlossenen Reaktorgehäuses (2) bis zur Oberfläche des das geschlossene Reaktorgehäuse (2) umgebenden Erdreichs erstreckt.

8. Biogasanlage nach einem der Ansprüche 1 bis 7, **dadurch gekennzeichnet, dass** das geschlossene Reaktorgehäuse (2) in das Erdreich eingelassen ist.

9. Biogasanlage nach einem der Ansprüche 1 bis 8, **dadurch gekennzeichnet, dass** im Erdreich unterhalb des geschlossenen Reaktorgehäuses (2) Drainagen (25) angeordnet sind.

10. Biogasanlage nach einem der Ansprüche 1 bis 9, **dadurch gekennzeichnet, dass** die Heizeinrichtung am Boden des Reaktionsbehälters (3) vorgesehen ist.

11. Biogasanlage nach einem der Ansprüche 1 bis 10, **dadurch gekennzeichnet, dass** in dem Reaktionsbehälter (3) oberhalb des organischen Materials eine Abdeckisolierung vorgesehen ist.

12. Biogasanlage nach einem der Ansprüche 1 bis 11, **dadurch gekennzeichnet, dass** am Boden und/oder in der Seitenwand des Reaktionsbehälters (3) eine Isolierschicht vorgesehen ist.

## Claims

1. Biogas facility having
- a closed reactor housing (2),
- a mixing, fermentation and pre-supply vessel (11) adjoining the inner wall surface (8) inside the reactor housing (2) and being separated from the intermediate region (6) by an intermediate wall (12) and connected to a feed device (13) for fresh organic material and to an inlet device (4),
- a reaction vessel (3) disposed in the reactor housing (2) at a distance from the inner wall surface (8) thereof, at which reaction vessel an inlet device (4) for pre-acidified organic material issues and to which reaction vessel a discharge device (5) to discharge gas-generating organic material from the reaction vessel (3) is attached,
- a separating wall (9) extending between the outer wall surface (7) of the reaction vessel (3) and the inner wall surface (8) of the reactor housing (2), and
- a heating device provided in the reaction vessel (3), wherein
- the discharge device (5) is formed in such a way that it introduces the gas-generating organic material from the reaction vessel (3) on one side of the separating wall (9) into the intermediate region (6) between the outer wall surface (7) of the reaction vessel (3) and the inner wall surface (8) of the reactor housing (2),
- the inlet device (4) and the discharge device (5) of the reaction vessel (3) are formed in a tubular manner, wherein the inlet device (4) passes through the discharge device (5) leaving a radial spacing, and
- in the reactor housing (2) in the region which adjoins the other side of the separating wall (9) an outlet device (10) for digested organic material is provided.

2. Biogas facility as claimed in claim 1, **characterised in that** the mixing, fermentation and pre-supply vessel (11) is fitted with a circulating screw.

3. Biogas facility as claimed in claim 1 or 2, **characterised in that** the connection of the discharge device (5) to the reaction vessel (3) and the point where the inlet device (4) issues into the reaction vessel (3) lie in a substantially diametrically opposite manner with respect to the middle axis of the reaction vessel (3).

4. Biogas facility as claimed in any one of claims 1 to 3, **characterised in that** in the closed reactor housing (2) gas-tight sheeting (14) is disposed extending over the reaction vessel (3) and the intermediate region (6).

5. Biogas facility as claimed in any one of claims 1 to 4, **characterised in that** the closed reactor housing (2) is closed at the top by a roof (15) and an insulating intermediate cover (16) spaced apart from the roof (15) is disposed below the roof (15).

6. Biogas facility as claimed in claim 5, **characterised in that** a fan (17) is disposed in the insulating intermediate cover (16).

7. Biogas facility as claimed in claim 5 or 6, **characterised in that** the roof (15) of the closed reactor housing (2) extends as far as the surface of the ground surrounding the closed reactor housing (2).

8. Biogas facility as claimed in any one of claims 1 to 7, **characterised in that** the closed reactor housing (2) is let into the ground.

9. Biogas facility as claimed in any one of claims 1 to 8, **characterised in that** drains (25) are disposed in the ground below the closed reactor housing (2).

10. Biogas facility as claimed in any one of claims 1 to 9, **characterised in that** the heating device is provided on the bottom of the reaction vessel (3).

11. Biogas facility as claimed in any one of claims 1 to 10, **characterised in that** covering insulation is provided in the reaction vessel (3) above the organic material.

12. Biogas facility as claimed in any one of claims 1 to 11, **characterised in that** an insulating layer is provided on the bottom and/or in the side wall of the reaction vessel (3).

## Revendications

1. Installation à biogaz comprenant
- un boîtier de réacteur (2) fermé,
- un réservoir de mélange, de fermentation et de pré-alimentation (11) adjacent à la surface de la paroi intérieure (8) dans le boîtier de réacteur (2), ce réservoir étant séparé de la partie intermédiaire (6) par une paroi intermédiaire (12) et communiquant avec un dispositif de chargement (13) pour une matière organique fraîche et avec un dispositif d'alimentation (4),
- un réservoir de réaction (3) disposé dans le boîtier de réacteur (2) à distance de la surface de la paroi intérieure (8) du boîtier de réacteur, sur lequel réservoir de réaction débouche un dispositif d'alimentation (4) pour une matière organique préacidifiée et auquel est raccordé un dispositif de décharge (5) pour un déchargement d'une matière organique gazeuse à partir du réservoir de réaction (3),
- une paroi de séparation (9) s'étendant entre la surface de la paroi extérieure (7) du réservoir de réaction (3) et la surface de la paroi intérieure (8) du boîtier de réacteur (2) et
- un dispositif de chauffage prévu dans le boîtier de réaction (3),
- le dispositif de décharge (5) étant exécuté de telle manière qu'il place la matière organique gazeuse provenant du réservoir de réaction (3) sur un côté de la paroi de séparation (9) dans la partie intermédiaire (6) entre la surface de la paroi extérieure (7) du réservoir de réaction (3) et la surface de la paroi intérieure (8) du boîtier de réacteur (2),
- le dispositif d'alimentation (4) et le dispositif de décharge (5) du réservoir de réaction (3) étant exécutés en forme de tube, le dispositif d'alimentation (4) passant à travers le dispositif de décharge (5) avec écart radial, et
- un dispositif de sortie (10) pour la matière organique putréfiée étant prévu dans le boîtier de réacteur (2) dans la partie qui est adjacente à l'autre côté de la paroi de séparation (9).

2. Installation à biogaz selon la revendication 1, **caractérisée en ce que** le réservoir de mélange, de fermentation et de pré-alimentation (11) est équipé d'une vis sans fin de recyclage.

3. Installation à biogaz selon la revendication 1 ou 2, **caractérisée en ce que** le raccordement du dispositif de décharge (5) au réservoir de réaction (3) et l'embouchure du dispositif d'alimentation (4) dans le réservoir de réaction (3) sont essentiellement diamétralement opposés par rapport à l'axe médian du réservoir de réaction (3).

4. Installation à biogaz selon l'une quelconque des revendications 1 à 3, **caractérisée en ce qu'**une feuille (14) étanche au gaz et s'étendant sur le réservoir de réaction (5) et la partie intermédiaire (6) est disposée dans le boîtier de réacteur (2) fermé.

5. Installation à biogaz selon l'une quelconque des revendications 1 à 4, **caractérisée en ce que** le boîtier de réacteur (2) fermé est fermé vers le haut par un toit (15) et **en ce qu'**un plafond intermédiaire isolant (16) est disposé en dessous du toit (15) à écart du toit (15).

6. Installation à biogaz selon la revendication 5, **caractérisée en ce qu'**un ventilateur (17) est disposé dans le plafond intermédiaire isolant (16).

7. Installation à biogaz selon la revendication 5 ou 6, **caractérisée en ce que** le toit (15) du boîtier de réacteur (2) fermé s'étend jusqu'à la surface du sol entourant le boîtier de réacteur (2) fermé.

8. Installation à biogaz selon l'une quelconque des revendications 1 à 7, **caractérisée en ce que** le boîtier de réacteur (2) fermé est encastré dans le sol.

9. Installation à biogaz selon l'une quelconque des revendications 1 à 8, **caractérisée en ce que** des drains (25) sont disposés dans le sol en dessous du boîtier de réacteur (2) fermé.

10. Installation à biogaz selon l'une quelconque des revendications 1 à 9, **caractérisée en ce que** le dispositif de chauffage est prévu au fond du réservoir de réaction (3).

11. Installation à biogaz selon l'une quelconque des revendications 1 à 10, **caractérisée en ce qu'**une isolation de recouvrement est prévue dans le réservoir de réaction (3) au-dessus de la matière organique.

12. Installation à biogaz selon l'une quelconque des revendications 1 à 11, **caractérisée en ce qu'**une couche isolante est prévue au fond et/ou dans la paroi latérale du réservoir de réaction (3).
